# EUROPÄISCHE PATENTSCHRIFT

(11) **EP 1 358 876 B1**
(45) Veröffentlichungstag und Bekanntmachung des Hinweises auf die Patenterteilung: **07.11.2007**
(21) Anmeldenummer: 02009719.2
(22) Anmeldetag: 30.04.2002
(51) Int. Cl.: A61K 9/50, A61K 31/704, A61P 17/10, A61K 36/00

(54) **Mikrokapseln mit Anti-Aknewirkstoffen**
Microcapsules containing anti-acne agents
Microcapsules contenant des actifs antiacneiques

(43) Veröffentlichungstag der Anmeldung: 05.11.2003
(73) Patentinhaber: Cognis IP Management GmbH, 40589 Düsseldorf (DE)
(72) Erfinder: Arias, Carmen, 08190 Sant Cugat del Vallés (ES); Rull Prous, Santiago, 08034 Barcelona (ES); Fabry, Bernd, Dr., 41352 Korschenbroich (DE)

(56) Entgegenhaltungen:
- EP-A- 1 064 910
- EP-A- 1 064 911
- EP-A- 1 064 912
- EP-A- 1 064 913
- ES-A- 2 137 125
- US-A- 5 679 374

## Beschreibung

### Gebiet der Erfindung

Die Erfindung befindet sich auf dem Gebiet der botanischen Extrakte und betrifft Mikrokapseln mit den Wirkstoffen der *Glycyrrhiza glabra,* Verfahren zur deren Herstellung sowie deren Verwendung zu Herstellung von Medikamenten zur Bekämpfung von Akne.

### Stand der Technik

Unter dem Begriff Akne versteht der Fachmann eine Hauterkrankung, die sich durch entzündliche und nicht entzündliche Knötchen auszeichnen und die zur Bildung von Pusteln, Abszessen und schließlich Narben führen kann. Obschon die Ursachen unterschiedlich sind, lässt sich Akne letztlich auf verstopfte Haarfollikel (Komedone) zurückführen. Neben einer hormonell bedingten Verstopfung der Haarfollikel-Mündungen durch Körperfette, besteht eine wesentliche Ursache für die Entstehung von Akne in der Entwicklung gewebeschädigender freier Fettsäuren und Enzyme durch Bakterien, wie beispielsweise Propionibacterium acnes.

Aus dem Stand der Technik sind eine ganze Reihe von Wirkstoffen bekannt, die zur Bekämpfung von Akne mit mehr oder minder großem Erfolg eingesetzt werden können. So sind beispielsweise aus dem EP 0661036 B1 (L'Oréal) Anti-Aknemittel in Form von Lipidvesikeln bekannt, die als Wirkstoff Azelainsäure enthalten. Gegenstand der Spanischen Patentschrift ES 2137125 A1 (Vinyals) die Verwendung des Zinksalzes der Glyzyrrhetinsäure gegen Akne. Von Nachteil ist jedoch, dass die Wirkstoffe üblicherweise in Salben und Tinkturen aufgelöst oder dispergiert werden müssen, was zu einem photochemischen Abbau oder Veränderung führen kann. Im übrigen wirken sie zwar gegen die die Akne auslösenden Mikroorganismen, unterstützen den Heilungsprozess jedoch nicht.

Die Aufgabe der Erfindung hat daher darin bestanden, eine neue Anbietungsform von Glyzyrrhetinsäure und deren Derivaten zur Verfügung zu finden, die gleichzeitig eine einfache Anwendung ermöglichen, die Stabilität der Wirkstoffe gegenüber Lagereinflüssen erhöhen und zudem den Heilungsprozess vorzugsweise durch anti-inflammatorische Eigenschaften unterstützen.

### Beschreibung der Erfindung

Gegenstand der Erfindung sind neue Mikrokapseln mit mittleren Durchmessern im Bereich von 0,0001 bis 5 mm, bestehend aus einer Hüllmembran und einer die Wirkstoffe enthaltenden Matrix, die dadurch erhältlich sind, dass man
(a1) aus Gelbildnern, Chitosanen und Glyzyrrhetinsäure und/oder deren Derivaten eine Matrix zubereitet,
(a2) gegebenenfalls die Matrix in einer Ölphase dispergiert,
(a3) die gegebenenfalls dispergierte Matrix mit wässrigen Lösungen anionischer Polymere behandelt und gegebenenfalls dabei die Ölphase entfernt.
oder
(b1) aus Gelbildnern, anionischen Polymeren und Glyzyrrhetinsäure und/oder deren Derivaten eine Matrix zubereitet,
(b2) gegebenenfalls die Matrix in einer Ölphase dispergiert,
(b3) die gegebenenfalls dispergierte Matrix mit wässrigen Chitosanlösungen behandelt und gegebenenfalls dabei die Ölphase entfernt.
oder
(c1) wässrige Zubereitungen von Glyzyrrhetinsäure und/oder deren Derivaten mit Ölkörpern in Gegenwart von Emulgatoren zu O/W-Emulsionen verarbeitet,
(c2) die so erhaltenen Emulsionen mit wässrigen Lösungen anionischer Polymere behandelt,
(c3) die so erhaltene Matrix mit wässrigen Chitosanlösungen in Kontakt bringt und
(c4) die so erhaltenen Verkapselungsprodukte von der wässrigen Phase abtrennt.

Die Mikrokapseln erweisen sich nicht nur in den Anwendung als außerordentlich leicht zu handhaben, überraschenderweise wurde auch gefunden, dass diese Darreichungsform den Heilungsprozess selbst fördert, da die in der Kapselhülle enthaltenen kationischen Biopolymeren selbst über eine anti-inflammatorische Wirkung verfügen. Ein weiterer Vorteil besteht darin, dass die verkapselten Wirkstoffe gegenüber Oxidationsprozessen stabilisiert werden, was die Lagerstabilität wesentlich verbessert.

Ein weiterer Gegenstand der Erfindung betrifft ein analoges Verfahren zur Herstellung von Mikrokapseln mit mittleren Durchmessern im Bereich von 0,0001 bis 5 mm, bestehend aus einer Hüllmembran und einer die Wirkstoffe enthaltenden Matrix, bei dem man
(a1) aus Gelbildnern, Chitosanen und Glyzyrrhetinsäure und/oder deren Derivaten eine Matrix zubereitet,
(a2) gegebenenfalls die Matrix in einer Ölphase dispergiert,
(a3) die gegebenenfalls dispergierte Matrix mit wässrigen Lösungen anionischer Polymere behandelt und gegebenenfalls dabei die Ölphase entfernt.
oder
(b1) aus Gelbildnern, anionischen Polymeren und Glyzyrrhetinsäure und/oder deren Derivaten eine Matrix zubereitet,
(b2) gegebenenfalls die Matrix in einer Ölphase dispergiert,
(b3) die gegebenenfalls dispergierte Matrix mit wässrigen Chitosanlösungen behandelt und gegebenenfalls dabei die Ölphase entfernt.
oder
(c1) wässrige Zubereitungen von Glyzyrrhetinsäure und/oder deren Derivaten mit Ölkörpern in Gegenwart von Emulgatoren zu O/W-Emulsionen verarbeitet,
(c2) die so erhaltenen Emulsionen mit wässrigen Lösungen anionischer Polymere behandelt,
(c3) die so erhaltene Matrix mit wässrigen Chitosanlösungen in Kontakt bringt und
(c4) die so erhaltenen Verkapselungsprodukte von der wässrigen Phase abtrennt.

### Glyzyrrhetinsäure und deren Derivate

Glyzyrrhetinsäure (s. Abbildung) und deren Derivate, die als Anti-Aknewirkstoffe im Sinne der Erfindung eingesetzt werden, finden sich als Komponenten in Extrakten der *Glycyrrhiza glabra* und sind für den Lakritzgeschmack verantwortlich.

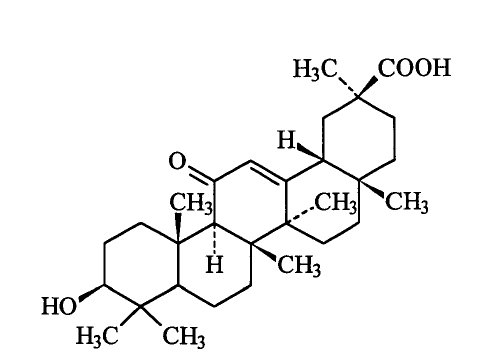

Im Sinne der Erfindung ist es aber nicht nur möglich, dass die reine Säure zum Einsatz gelangt, alternativ oder in Abmischung kommen auch die Alkali-, Erdalkali-, Ammonium-, Alkylammonium-, Alkanolammonium-, Glucammonium- und Zinksalzen der Glyzyrrhetinsäure, die Ester der Glyzyrrhetinsäure mit linearen oder verzweigten aliphatischen Alkoholen mit 1 bis 18 Kohlenstoffatomen sowie die Voll- oder Partialester der Glyzyrrhetinsäure mit Polyolen mit 2 bis 15 Kohlenstoffatomen und mindestens 2 Hydroxylgruppen in Frage.

Typische Beispiele sind die Natrium-, Kalium-, Ammonium-, Triethanolammonium-, Glucammonium und Zinksalze der Glyzyrrhetinsäure, die Ester der Glyzyrrhetinsäure mit Methanol, Ethanol, den isomeren Propanolen und Butanolen sowie Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol und deren Gemischen. Weitere Beispiele sind die Voll- oder Partialester der Glyzyrrhetinsäure mit Glycerin; Alkylenglycolen, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton; technischen Oligoglyceringemischen mit einem Eigenkondensationsgrad von 1,5 bis 10 wie etwa technische Diglyceringemische mit einem Diglyceringehalt von 40 bis 50 Gew.-%; Methyolverbindungen, wie insbesondere Trimethylolethan, Trimethylolpropan, Trimethylolbutan, Pentaerythrit und Dipentaerythrit; Niedrigalkylglucosiden, insbesondere solche mit 1 bis 8 Kohlenstoffen im Alkylrest, wie beispielsweise Methyl- und Butylglucosid; Zuckeralkoholen mit 5 bis 12 Kohlenstoffatomen, wie beispielsweise Sorbit oder Mannit; Zuckern mit 5 bis 12 Kohlenstoffatomen, wie beispielsweise Glucose oder Saccharose; Aminozuckern, wie beispielsweise Glucamin; oder Dialkoholaminen, wie Diethanolamin oder 2-Amino-1,3-propandiol. Vorzugsweise setzt man als Komponente (b) das Kalium-, Ammonium- und/oder Zinksalz der Glyzyrrhetinsäure oder deren Ester mit Fettalkoholen mit 16 bis 18 Kohlenstoffatomen ein.

Anstelle der reinen Glyzyrrhetinsäure bzw. deren Derivate kann in einer bevorzugten Ausführungsform der vorliegenden Erfindung der Extrakt der *Glycyrrhiza glabra* direkt eingesetzt werden, welcher üblicherweise eine Mischung von 18-β-Glyzyrrhetinsäure, 18-β-Glyzyrrhetinsäure-Kaliumsalz und 18-β-Glyzyrrhetinsäurestearat darstellt und in einer Reinheit von 98 Gew.-% beispielsweise unter der Marke Plantactiv® GLA 18 (Cognis) im Handel erhältlich ist. Die Glyzyrrhetinsäure und/oder ihre Derivate können in den Mikrokapseln in Mengen von 1 bis 30 Gew.-% - bezogen auf das Kapselgewicht - vorhanden sein.

### Weitere Wirkstoffe

Als weitere Komponenten können die erfindungsgemäßen Mikrokapseln andere gegen Akne wirksame oder anti-inflammatorische Stoffe enthalten, wie beispielsweise Pflanzenextrakte oder Vitamin B6. Typische Beispiele für geeignete Pflanzenextrakte sind die Wirkstoffe folgender Pflanzen : *Aesculus hippocastanum* (Rosskastanie), *Argania spinosa, Babtisia tinctoria* (wilder Indigo), *Centella asiatica, Camellia sinensis* (Grüner Tee), *Chamomilla recutita* (Kamille), *Ginkgo biloba* (Ginkgo), *Olea europea* (Olive), *Litschi chinensis* (Litchi), *Melissa officinalis* (Zitronenmelisse), *Panax ginseng* (Ginseng), *Passiflora incarnata* (Passionsblume), *Prunus dulcis* (Süßmandel), *Pterocarpus marsupium, Ruscus aculeatus, Trifolium pratense* (Red Clover), *Uva ursi* (Bärtraube), *Vaccinium myrtillus* (Blaubeere), *Vigna aconitifolia*, und *Vitis vinifera* (wilder Wein). Die Glyzyrrhetinsäure bzw. deren Derivate und die weiteren Wirkstoffe können im Gewichtsverhältnis 90 : 10 bis 10 : 90, vorzugsweise 75 : 25 bis 25 : 75 und insbesondere 60 : 40 bis 40 : 60 enthalten sein.

### Mikrokapseln

Unter dem Begriff "Mikrokapsel" werden vom Fachmann sphärische Aggregate mit einem Durchmesser im Bereich von etwa 0,0001 bis etwa 5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden geschmolzene Wachse in einer Matrix aufgenommen ("microsponge"), die als Mikropartikel zusätzlich mit filmbildenden Polymeren umhüllt sein können. Die mikroskopisch kleinen Kapseln, auch Nanokapseln genannt, lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccaride, wie Stärke oder Dextran, Polypeptide, Proteinghydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon.

Beispiele für Mikrokapseln des Stands der Technik sind folgende Handelsprodukte (in Klammern angegeben ist jeweils das Hüllmaterial) : *Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropylmethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide) sowie *Primaspheres* und *Primasponges* (Chitosan, Alginate) und *Primasys* (Phospholipide). Chitosanmikrokapseln und Verfahren zu ihrer Herstellung sind Gegenstand früherer Patenanmeldungen der Patentanmelderin [WO 01/01926, WO 01/01927, WO 01/01928, WO 01/01929].

### Gelbildner

Im Sinne der Erfindung werden als Gelbildner vorzugsweise solche Stoffe in Betracht gezogen, welche die Eigenschaft zeigen in wässriger Lösung bei Temperaturen oberhalb von 40 °C Gele zu bilden. Typische Beispiele hierfür sind Heteropolysaccharide und Proteine. Als thermogelierende Heteropolysaccharide kommen vorzugsweise Agarosen in Frage, welche in Form des aus Rotalgen zu gewinnenden Agar-Agar auch zusammen mit bis zu 30 Gew.-% nicht-gelbildenden Agaropektinen vorliegen können. Hauptbestandteil der Agarosen sind lineare Polysaccharide aus D-Galaktose und 3,6-Anhydro-L-galaktose, die alternierend β-1,3- und β-1,4-glykosidisch verknüpft sind. Die Heteropolysaccharide besitzen vorzugsweise ein Molekulargewicht im Bereich von 110.000 bis 160.000 und sind sowohl farb- als auch geschmacklos. Als Alternativen kommen Pektine, Xanthane (auch Xanthan Gum) sowie deren Mischungen in Frage. Es sind weiterhin solche Typen bevorzugt, die noch in 1-Gew.-%iger wässriger Lösung Gele bilden, die nicht unterhalb von 80 °C schmelzen und sich bereits oberhalb von 40 °C wieder verfestigen. Aus der Gruppe der thermogelierenden Proteine seien exemplarisch die verschiedenen Gelatine-Typen genannt.

### Chitosane

Chitosane stellen Biopolymere dar und werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein enthalten:

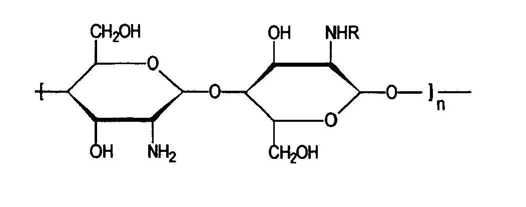

Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt. Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren, das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Vorzugsweise werden solche Typen eingesetzt, wie die ein durchschnittliches Molekulargewicht von 10.000 bis 500.000 bzw. 800.000 bis 1.200.000 Dalton aufweisen und/oder eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% besitzen. Aus Gründen der besseren Wasserlöslichkeit werden die Chitosane in der Regel in Form ihrer Salze, vorzugsweise als Glycolate eingesetzt.

### Ölphase

Die Matrix kann vor der Bildung der Membran optional in einer Ölphase dispergiert werden. Als Öle kommen für diesen Zweck beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglycerid-mischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe, wie z.B. wie Squalan, Squalen oder Dialkylcyclohexane in Betracht.

### Anionpolymere

Die anionische Polymere haben die Aufgabe, mit den Chitosanen Membranen zu bilden. Für diesen Zweck eignen sich vorzugsweise Salze der Alginsäure. Bei der Alginsäure handelt es sich um ein Gemisch carboxylgruppenhaltiger Polysaccharide mit folgendem idealisierten Monomerbaustein:

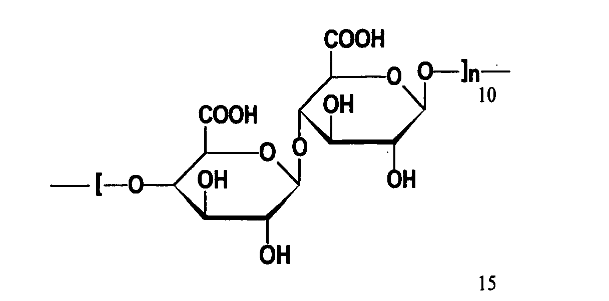

Das durchschnittliche Molekulargewicht der Alginsäuren bzw. der Alginate liegt im Bereich von 150.000 bis 250.000. Dabei sind als Salze der Alginsäure sowohl deren vollständige als auch deren partiellen Neutralisationsprodukte zu verstehen, insbesondere die Alkalisalze und hierunter vorzugsweise das Natriumalginat ("Algin") sowie die Ammonium- und Erdalkalisalze. besonders bevorzugt sind Mischalginate, wie z.B. Natrium/Magnesium- oder Natrium/Calciumalginate. In einer alternativen Ausführungsform der Erfindung kommen für diesen Zweck jedoch auch anionische Chitosanderivate, wie z.B. Carboxylierungs- und vor allem Succinylierungsprodukte in Frage. Alternativ kommen auch Poly(meth)acrylate mit durchschnittlichen Molekulargewichten im Bereich von 5.000 bis 50.000 Dalton sowie die verschiedenen Carboxymethylcellulosen in Frage. Anstelle der anionischen Polymeren können für die Ausbildung der Hüllmembran auch anionische Tenside oder niedermolekulare anorganische Salze, wie beispielsweise Pyrophosphate eingesetzt werden.

### Emulgatoren

Als Emulgatoren kommen beispielsweise nichtionogene Tenside aus mindestens einer der folgenden Gruppen in Frage:
➢ Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
➢ Alkyl- und/oder Alkenyloligoglykoside mit 8 bis 22 Kohlenstoffatomen im Alk(en)ylrest und deren ethoxylierte Analoga;
➢ Anlagerungsprodukte von 1 bis 15 Mol Ethylenoxid an Ricinusöl und/oder gehärtetes Ricinusöl;
➢ Anlagerungsprodukte von 15 bis 60 Mol Ethylenoxid an Ricinusöl und/oder gehärtetes Ricinusöl;
➢ Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
➢ Mono-, Di- und Trialkylphosphate sowie Mono-, Di- und/oder Tri-PEG-alkylphosphate und deren Salze;
➢ Wollwachsalkohole;
➢ Polysiloxan-Polyalkyl-Polyether-Copolymere bzw. entsprechende Derivate;
➢ Block-Copolymere z.B. Polyethylenglycol-30 Dipolyhydroxystearate;
➢ Polymeremulgatoren, z.B. Pemulen-Typen (TR-1,TR-2) von Goodrich;
➢ Polyalkylenglycole sowie
➢ Glycerincarbonat.

### ➢ Ethylenoxidanlagerungsprodukte

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind als Rückfettungsmittel für kosmetische Zubereitungen bekannt.

### ➢ Alkyl- und/oder Alkenyloligoglykoside

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

### ➢ Partialglyceride

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

### ➢ Sorbitanester

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

### ➢ Polyglycerinester

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

### ➢ Anionische Emulgatoren

Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

### ➢ Amphotere und kationische Emulgatoren

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOHoder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### Herstellverfahren Mikrokapseln

Zur Herstellung der Mikrokapseln stellt man üblicherweise eine 1 bis 10, vorzugsweise 2 bis 5 Gew.-%ige wässrige Lösung des Gelbildners, vorzugsweise des Agar-Agars her und erhitzt diese unter Rückfluss. In der Siedehitze, vorzugsweise bei 80 bis 100°C, wird eine zweite wässrige Lösung zugegeben, welche das Chitosan in Mengen von 0,1 bis 2, vorzugsweise 0,25 bis 0,5 Gew.-% und die Wirkstoffe in Mengen von 0,1 bis 25 und insbesondere 0,25 bis 10 Gew.-% enthält; diese Mischung wird als Matrix bezeichnet. Die Beladung der Mikrokapseln mit Wirkstoffen kann daher ebenfalls 0,1 bis 25 Gew.-% bezogen auf das Kapselgewicht betragen. Falls gewünscht, können zu diesem Zeitpunkt zur Viskositätseinstellung auch wasserunlösliche Bestandteile, beispielsweise anorganische Pigmente zugegeben werden, wobei man diese in der Regel in Form von wässrigen oder wässrig/alkoholischen Dispersionen zusetzt. Zur Emulgierung bzw. Dispergierung der Wirkstoffe kann es ferner von Nutzen sein, der Matrix Emulgatoren und/oder Lösungsvermittler hinzuzugeben. Nach der Herstellung der Matrix aus Gelbildner, Chitosan und Wirkstoffen kann die Matrix optional in einer Ölphase unter starker Scherung sehr fein dispergiert werden, um bei der nachfolgenden Verkapselung möglichst kleine Teilchen herzustellen. Dabei hat es sich als besonders vorteilhaft erwiesen, die Matrix auf Temperaturen im Bereich von 40 bis 60 °C zu erwärmen, während man die Ölphase auf 10 bis 20 °C kühlt. Im letzten, nun wieder obligatorischen Schritt erfolgt dann die eigentliche Verkapselung, d.h. die Ausbildung der Hüllmembran durch Inkontaktbringen des Chitosans in der Matrix mit den anionischen Polymeren. Hierzu empfiehlt es sich, die gegebenenfalls in der Ölphase dispergierte Matrix bei einer Temperatur im Bereich von 40 bis 100, vorzugsweise 50 bis 60 °C mit einer wässrigen, etwa 1 bis 50 und vorzugsweise 10 bis 15 Gew.-%ige wässrigen Lösung des Anionpolymers zu behandeln und dabei - falls erforderlich - gleichzeitig oder nachträglich die Ölphase zu entfernen. Die dabei resultierenden wässrigen Zubereitungen weisen in der Regel einen Mikrokapselgehalt im Bereich von 1 bis 10 Gew.-% auf. In manchen Fällen kann es dabei von Vorteil sein, wenn die Lösung der Polymeren weitere Inhaltsstoffe, beispielsweise Emulgatoren oder Konservierungsmittel enthält. Nach Filtration werden Mikrokapseln erhalten, welche im Mittel einen Durchmesser im Bereich von vorzugsweise etwa 1 mm aufweisen. Es empfiehlt sich, die Kapseln zu sieben, um eine möglichst gleichmäßige Größenverteilung sicherzustellen. Die so erhaltenen Mikrokapseln können im herstellungsbedingten Rahmen eine beliebige Form aufweisen, sie sind jedoch bevorzugt näherungsweise kugelförmig. Alternativ kann man die Anionpolymere auch zur Herstellung der Matrix einsetzen und die Verkapselung mit den Chitosanen durchführen.

In einem alternativen Verfahren wird zur Herstellung der erfindungsgemäßen Mikrokapseln wird zunächst eine O/W-Emulsion zubereitet, welche neben dem Ölkörper, Wasser und den Wirkstoffen eine wirksame Menge Emulgator enthält. Zur Herstellung der Matrix wird diese Zubereitung unter starkem Rühren mit einer entsprechenden Menge einer wäßrigen Anionpolymerlösung versetzt. Die Membranbildung erfolgt durch Zugabe der Chitosanlösung. Der gesamte Vorgang findet vorzugsweise im schwach sauren Bereich bei pH = 3 bis 4 statt. Falls erforderlich erfolgt die pH-Einstellung durch Zugabe von Mineralsäure. Nach der Membranbildung wird der pH-Wert auf 5 bis 6 angehoben, beispielsweise durch Zugabe von Triethanolamin oder einer anderen Base. Hierbei kommt es zu einem Anstieg der Viskosität, die durch Zugabe von weiteren Verdickungsmitteln, wie z.B. Polysacchariden, insbesondere Xanthan-Gum, Guar-Guar, Agar-Agar, Alginaten und Tylosen, Carboxymethylcellulose und Hydroxyethylcellulose, höhermolekularen Polyethylenglycolmono- und -diesten von Fettsäuren, Polyacrylaten, Polyacrylamiden und dergleichen noch unterstützt werden kann. Abschließend werden die Mikrokapseln von der wässrigen Phase beispielsweise durch Dekantieren, Filtrieren oder Zentrifugieren abgetrennt.

### Gewerbliche Anwendbarkeit

Die erfindungsgemäßen Mikrokapseln enthalten Glyzyrrhetinsäure und deren Derivate, die insbesondere gegenüber solchen Keimen wirksam sind, die für die Auslösung von Akne verantwortlich sind. Ein weiterer Gegenstand der Erfindung betrifft daher die Verwendung der erfindungsgemäßen Mikrokapseln zur Herstellung eines Medikamentes zur Bekämpfung von Akne, insbesondere von Acne vulgaris.

### Beispiele

**Beispiel 1.** In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan (Hydagen® DCMF, 1 Gew.-%ig in Glycolsäure, Cognis, Düsseldorf/FRG), 8 g Glyzyrrhetinsäure-Zinksalz, Phenonip® (Konservierungsmittelmischung enthaltend Phenoxyethanol und Parabene) und 0,5 g Polysorbat-20 (Tween® 20, ICI) in ad 100 g Wasser versetzt. Die erhaltene Matrix wurde filtriert, auf 60 °C erwärmt und in eine 0,5 Gew.-%ige Natriumalginatlösung getropft. Nach dem Sieben wurde eine wässrige Zubereitung erhalten, die 8 Gew.-% Mikrokapseln mit einem mittleren Durchmesser von 1 mm enthielt.

**Beispiel 2.** In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan (Hydagen® DCMF, 1 Gew.-%ig in Glycolsäure, Cognis, Düsseldorf/FRG), 8 g Glyzyrrhetinsäure-Zinksalz, 0,5 g Phenonip® (Konservierungsmittelmischung enthaltend Phenoxyethanol und Parabene) und 0,5 g Polysorbat-20 (Tween® 20, ICI) in ad 100 g Wasser versetzt. Die erhaltene Matrix wurde filtriert, auf 50 °C temperiert und unter starken Rühren im 2,5fachen Volumen Paraffinöl, das zuvor auf 15 °C gekühlt worden war, dispergiert. Die Dispersion wurde anschließend mit einer wässrigen Lösung enthaltend 1 Gew.-% Natriumlaurylsulfat und 0,5 Gew.-% Natriumalginat und dann mehrfach mit einer 0,5 Gew.-%igen wässrigen Phenoniplösung gewaschen, wobei die Ölphase entfernt wurde. Nach dem Sieben wurde eine wässrige Zubereitung erhalten, die 8 Gew.-% Mikrokapseln mit einem mittleren Durchmesser von 1 mm enthielt.

**Beispiel 3.** In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan (Hydagen® DCMF, 1 Gew.-%ig in Glycolsäure, Cognis, Düsseldorf/FRG), 6 g Glyzyrrhetinsäure-Zinksalz, 2 g Camellia sinensis-Extrakt, 0,5 g Phenonip® (Konservierungsmittelmischung enthaltend Phenoxyethanol und Parabene) und 0,5 g Polysorbat-20 (Tween® 20, ICI) in ad 100 g Wasser versetzt. Die erhaltene Matrix wurde filtriert, auf 60 °C erwärmt und in eine 15 Gew.-%ige Lösung von Sodium Laureth Sulfate getropft. Nach dem Sieben wurde eine wässrige Zubereitung erhalten, die 9 Gew.-% Mikrokapseln mit einem mittleren Durchmesser von 1 mm enthielt.

**Beispiel 4.** In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan (Hydagen® DCMF, 1 Gew.-%ig in Glycolsäure, Cognis, Düsseldorf/FRG), 8 g Glycyrrhiza glabra-Extrakt (Plantactiv® GLA 18, 98 Gew.-%ig, Cognis), 0,5 g Phenonip® (Konservierungsmittelmischung enthaltend Phenoxyethanol und Parabene) und 0,5 g Polysorbat-20 (Tween® 20, ICI) in ad 100 g Wasser versetzt. Die erhaltene Matrix wurde filtriert, auf 60 °C erwärmt und in eine 15 Gew.-%ige Lösung von Natriumpyrophosphat getropft. Nach dem Sieben wurde eine wässrige Zubereitung erhalten, die 8 Gew.-% Mikrokapseln mit einem mittleren Durchmesser von 1 mm enthielt.

**Beispiel 5.** In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan (Hydagen® DCMF, 1 Gew.-%ig in Glycolsäure, Cognis, Düsseldorf/FRG), 6 g Glycyrrhiza glabra-Extrakt (Plantactiv® GLA 18, 98 Gew.-%ig, Cognis), 2 g Camellia sinensis-Extrakt, 0,5 g Phenonip® (Konservienmgsmittelmischung enthaltend Phenoxyethanol und Parabene) und 0,5 g Polysorbat-20 (Tween® 20, ICI) in ad 100 g Wasser versetzt. Die so erhaltene Matrix wurde filtriert, auf 50 °C temperiert und unter starken Rühren im 2,5fachen Volumen Paraffinöl, das zuvor auf 15 °C gekühlt worden war, dispergiert. Die Dispersion wurde anschließend mit einer 15 Gew.-%igen Natriumpyrophosphatlösung und dann mehrfach mit einer 0,5 Gew.-%igen wässrigen Phenoniplösung gewaschen, wobei die Ölphase entfernt wurde. Nach dem Sieben wurde eine wässrige Zubereitung erhalten, die 10 Gew.-% Mikrokapseln mit einem mittleren Durchmesser von 1 mm enthielt.

**Beispiel 6.** In einer Rührapparatur wurden 0,5 g Konservierungsmittel (Phenonip®) in 50 g einer 2 Gew.-%igen wässrigen Zubereitung von Carboxymethylcellulose gelöst und die Mischung auf pH = 3,5 eingestellt. Anschließend wurde unter starkem Rühren eine Mischung bestehend aus 10 g Glyzyrrhetinsäure-Zinksalz und 0,5 g Sorbitanmonostearat+20EO (Eumulgin® SMS 20, Cognis Deutschland GmbH) hinzugegeben. Danach wurde unter weiterem Rühren eine solche Menge einer 1 Gew.-%igen Lösung von Chitosan in Glycolsäure (Hydagen® CMF Cognis Deutschland GmbH) hinzugegeben, dass sich eine Chitosankonzentration von 0,075 Gew.-% - bezogen auf die Zubereitung - einstellte. Anschließend wurde der pH-Wert durch Zugabe von Triethanolamin auf 5,5 angehoben und die entstandenen Mikrokapseln dekantiert.

**Beispiel 7.** In einer Rührapparatur wurden 0,5 g Konservierungsmittel (Phenonip®) in 50 g einer 2 Gew.-%igen wässrigen Zubereitung von Polyacrylsäure (Pemulen® TR-2) gelöst, wobei sich ein pH-Wert von 3 einstellte. Anschließend wurde unter starkem Rühren eine Mischung bestehend aus 10 g Glycyrrhiza glabra-Extrakt (Plantactiv® GLA 18, 98 Gew.-%ig, Cognis), und 0,5 g Sorbitanmonolaurat+15EO (Eumulgin® SML 15, Cognis Deutschland GmbH) hinzugegeben. Danach wurde unter weiterem Rühren eine solche Menge einer 1 Gew.-%igen Lösung von Chitosan in Glycolsäure (Hydagen® CMF Cognis Deutschland GmbH) hinzugegeben, dass sich eine Chitosankonzentration von 0,01 Gew.-% - bezogen auf die Zubereitung - einstellte. Anschließend wurde der pH-Wert durch Zugabe von Triethanolamin auf 5,5 angehoben und die entstandenen Mikrokapseln dekantiert.

## Patentansprüche

1. Mikrokapseln mit mittleren Durchmessern im Bereich von 0,0001 bis 5 mm, bestehend aus einer Hüllmembran und einer die Wirkstoffe enthaltenden Matrix, dadurch erhältlich, dass man
(a1) aus Gelbildnern, Chitosanen und Glyzyrrhetinsäure und/oder deren Derivaten eine Matrix zubereitet,
(a2) gegebenenfalls die Matrix in einer Ölphase dispergiert,
(a3) die gegebenenfalls dispergierte Matrix mit wässrigen Lösungen anionischer Polymere behandelt und gegebenenfalls dabei die Ölphase entfernt.
oder
(b1) aus Gelbildnern, anionischen Polymeren und Glyzyrrhetinsäure und/oder deren Derivaten eine Matrix zubereitet,
(b2) gegebenenfalls die Matrix in einer Ölphase dispergiert,
(b3) die gegebenenfalls dispergierte Matrix mit wässrigen Chitosanlösungen behandelt und gegebenenfalls dabei die Ölphase entfernt.
oder
(c1) wässrige Zubereitungen von Glyzyrrhetinsäure und/oder deren Derivaten mit Ölkörpern in Gegenwart von Emulgatoren zu O/W-Emulsionen verarbeitet,
(c2) die so erhabenen Emulsionen mit wässrigen Lösungen anionischer Polymere behandelt,
(c3) die so erhaltene Matrix mit wässrigen Chitosanlösungen in Kontakt bringt und
(c4) die so erhaltenen Verkapselungsprodukte von der wässrigen Phase abtrennt.

2. Verfahren zur Herstellung von Mikrokapseln mit mittleren Durchmessern im Bereich von 0,0001 bis 5 mm, bestehend aus einer Hüllmembran und einer die Wirkstoffe enthaltenden Matrix, bei dem man
(a1) aus Gelbildnern, Chitosanen und Glyzyrrhetinsäure und/oder deren Derivaten eine Matrix zubereitet,
(a2) gegebenenfalls die Matrix in einer Ölphase dispergiert,
(a3) die gegebenenfalls dispergierte Matrix mit wässrigen Lösungen anionischer Polymere behandelt und gegebenenfalls dabei die Ölphase entfernt.
oder
(b1) aus Gelbildnern, anionischen Polymeren und Glyzyrrhetinsäure und/oder deren Derivaten eine Matrix zubereitet,
(b2) gegebenenfalls die Matrix in einer Ölphase dispergiert,
(b3) die gegebenenfalls dispergierte Matrix mit wässrigen Chitosanlösungen behandelt und gegebenenfalls dabei die Ölphase entfernt.
oder
(c1) wässrige Zubereitungen von Glyzyrrhetinsäure und/oder deren Derivaten mit Ölkörpern in Gegenwart von Emulgatoren zu O/W-Emulsionen verarbeitet,
(c2) die so erhaltenen Emulsionen mit wässrigen Lösungen anionischer Polymere behandelt,
(c3) die so erhaltene Matrix mit wässrigen Chitosanlösungen in Kontakt bringt und
(c4) die so erhaltenen Verkapselungsprodukte von der wässrigen Phase abtrennt.

3. Verfahren nach Anspruch 2, **dadurch gekennzeichnet, dass** man Derivate der Glyzyrrhetinsäure einsetzt, die ausgewählt sind aus der Gruppe, die gebildet wird von Alkali- Erdalkali-, Ammonium-, Alkylammonium-, Alkanolammonium-, Glucammonium- und Zinksalzen der Glyzyrrhetinsäure, den Estern der Glyzyrrhetinsäure mit linearen oder verzweigten aliphatischen Alkoholen mit 1 bis 18 Kohlenstoffatomen sowie den Voll- oder Partialestem der Glyzyrrhetinsäure mit Polyolen mit 2 bis 15 Kohlenstoffatomen und mindestens 2 Hydroxylgruppen.

4. Verfahren nach den Ansprüchen 2 und/oder 3, **dadurch gekennzeichnet, dass** man als Derivate der Glyzyrrhetinsäure einen Extrakt der *Glycyrrhiza glabra* einsetzt.

5. Verfahren nach mindestens einem der Ansprüche 2 bis 4, **dadurch gekennzeichnet, dass** man als Derivate der Glyzyrrhetinsäure das Kalium-, Ammonium- und/oder Zinksalz oder deren Ester mit Fettalkoholen mit 16 bis 18 Kohlenstoffatomen einsetzt.

6. Verfahren nach mindestens einem der Ansprüche 2 bis 5, **dadurch gekennzeichnet, dass** man als weitere Wirkstoffe Pflanzenextrakte und/oder Vitamin B6 einsetzt.

7. Verfahren nach Anspruch 6, **dadurch gekennzeichnet, dass** man Extrakte von Pflanzen einsetzt, die ausgewählt sind aus der Gruppe, die gebildet wird von *Aesculus hippocastanum* (Rosskastanie), *Argania spinosa, Baptisia tinctoria* (wilder Indigo), *Centella asiatica, Camellia sinensis* (Grüner Tee), *Chamomilla recutita* (Kamille), *Ginkgo biloba* (Ginkgo), *Oleo euro*paea (Olive), *Litchi chinensis* (Litchi), *Melissa officinalis* (Zitronenmelisse), *Panax ginseng* (Ginseng), *Passiflora incarnata* (Passionsblume), *Prunus dulcis* (Süßmandel), *Pterocarpus marsupium, Ruscus aculeatus, Trifolium pratense* (Red Clover), *Uva ursi* (Bärtraube), *Vaccinium myrtillus* (Blaubeere), *Vigna aconitifolia,* und *Vitis vinifera* (wilder Wein) sowie deren Gemischen.

8. Verfahren nach den Ansprüchen 6 und/oder 7, **dadurch gekennzeichnet, dass** man die Glyzyrrhetinsäure und/oder deren Derivate und die Pflanzenextrakte und/oder das Vitamin B6 im Gewichtsverhältnis 90 : 10 bis 10 : 90 einsetzt.

9. Verfahren nach mindestens einem der Ansprüche 2 bis 8, **dadurch gekennzeichnet, dass** man als Gelbildner Heteropolysaccharide oder Proteine einsetzt.

10. Verfahren nach Anspruch 9, **dadurch gekennzeichnet, dass** man als Heteropolysaccharide Agarosen, Agar-Agar, Pektine, Xanthane sowie deren Gemische einsetzt.

11. Verfahren nach Anspruch 9, **dadurch gekennzeichnet, dass** man als Proteine Gelatine einsetzt.

12. Verfahren nach mindestens einem der Ansprüche 2 bis 11, **dadurch gekennzeichnet, dass** man Chitosane einsetzt, die ein mittleres Molekulargewicht im Bereich von 10.000 bis 500.000 bzw. 800.000 bis 1.200.000 Dalton aufweisen.

13. Verfahren nach mindestens einem der Ansprüche 2 bis 12, **dadurch gekennzeichnet, dass** man anionische Polymere einsetzt, die ausgewählt sind aus der Gruppe, die gebildet wird von Salzen der Alginsäure, anionischen Chitosanderivaten, Poly(meth)acrylaten und Carboxymethylcellulosen.

14. Verwendung von Mikrokapseln nach Anspruch 1 zur Herstellung eines Medikamentes zur Bekämpfung von Akne.

## Claims

1. Microcapsules with mean diameters of 0.0001 to 5 mm which consist of a membrane and a matrix containing the active components and which may be obtained by
(a1) preparing a matrix from gel formers, chitosans and glycyrrhetic acid and/or derivatives thereof,
(a2) optionally dispersing the matrix in an oil phase and
(a3) treating the dispersed matrix with aqueous solutions of anionic polymers and optionally removing the oil phase in the process
or
(b1) preparing a matrix from gel formers, anionic polymers and glycyrrhetic acid and/or derivatives thereof,
(b2) optionally dispersing the matrix in an oil phase and
(b3) treating the dispersed matrix with aqueous chitosan solutions and optionally removing the oil phase in the process
or
(c1) processing aqueous preparations of glycyrrhetic acid and/or derivatives thereof with oil components in the presence of emulsifiers to form o/w emulsions,
(c2) treating the emulsions thus obtained with aqueous solutions of anionic polymers,
(c3) contacting the matrix thus obtained with aqueous chitosan solutions and
(c4) removing the encapsulated products thus obtained from the aqueous phase.

2. A process for the production of microcapsules with mean diameters of 0.0001 to 5 mm which consist of a membrane and a matrix containing the active components, **characterized in that** it comprises the steps of
(a1) preparing a matrix from gel formers, chitosans and glycyrrhetic acid and/or derivatives thereof,
(a2) optionally dispersing the matrix in an oil phase and
(a3) treating the dispersed matrix with aqueous solutions of anionic polymers and optionally removing the oil phase in the process
or
(b1) preparing a matrix from gel formers, anionic polymers and glycyrrhetic acid and/or derivatives thereof,
(b2) optionally dispersing the matrix in an oil phase and
(b3) treating the dispersed matrix with aqueous chitosan solutions and optionally removing the oil phase in the process
or
(c1) processing aqueous preparations of glycyrrhetic acid and/or derivatives thereof with oil components in the presence of emulsifiers to form o/w emulsions,
(c2) treating the emulsions thus obtained with aqueous solutions of anionic polymers,
(c3) contacting the matrix thus obtained with aqueous chitosan solutions and
(c4) removing the encapsulated products thus obtained from the aqueous phase.

3. A process as claimed in claim 2, **characterized in that** glycyrrhetic acid derivatives selected from the group consisting of alkali metal, alkaline earth metal, ammonium, alkylammonium, alkanolammonium, glucammonium and zinc salts of glycyrrhetic acid, esters of glycyrrhetic acid with linear or branched aliphatic C₁₋₁₈ alcohols and the full or partial esters of glycyrrhetic acid with polyols containing 2 to 15 carbon atoms and at least 2 hydroxyl groups are used.

4. A process as claimed in claims 2 and/or 3, **characterized in that** an extract of *Glycyrrhiza glabra* is used as component (b).

5. A process as claimed in at least one of claims 2 to 4, **characterized in that** the potassium, ammonium and/or zinc salt of glycyrrhetic acid or esters thereof with C₁₆₋₁₈ fatty alcohols are used as the glycyrrhetic acid derivatives.

6. A process as claimed in at least one of claims 2 to 5, **characterized in that** plant extracts and/or vitamin B6 is/are used as additional active component(s).

7. A process as claimed in claim 6, **characterized in that** extracts of plants selected from the group consisting of *Aesculus hippocastanum* (horse chestnut), *Argania spinosa, Babtista tinctoria* (wild indigo), *Cantella asiatica, Camelilla sinensis* (green tea), *Chamonella recutita* (chamomile), *Gingko biloba* (gingko), *Oleo europa* (olive), *Litschi chinensis* (lychee), *Melissa officinalis* (lemon melissa), *Panax ginseng* (ginseng), *Passiflora incarnata* (passion flower), *Prunus dulcis* (sweet almond), *Pterocarpus marsupium, Ruscus aculeatus, Trifolium pratense* (red clover), *Uva ursi* (bearberry), *Vaccinium myrtillus* (blueberry), *Vigna acontifolia* and *Vitis vinifera* (wild vine) and mixtures thereof are used.

8. A process as claimed in claims 6 and/or 7, **characterized in that** the glycyrrhetic acid and/or derivatives thereof and the plant extracts and/or the vitamin B6 are used in a ratio by weight of 90:10 to 10:90.

9. A process as claimed in at least one of claims 2 to 8, **characterized in that** heteropolysaccharides or proteins are used as the gel former.

10. A process as claimed in claim 9, **characterized in that** agaroses, agar agar, pectins, xanthans and mixture thereof are used as the heteropolysaccharides.

11. A process as claimed in claim 9, **characterized in that** gelatin is used as the protein.

12. A process as claimed in at least one of claims 2 to 11, **characterized in that** chitosans with an average molecular weight of 10,000 to 500,000 or 800,000 to 1,200,000 dalton are used.

13. A process as claimed in at least one of claims 2 to 12, **characterized in that** anionic polymers selected from the group consisting of salts of alginic acid, anionic chitosan derivatives, poly(meth)acrylates and carboxymethyl celluloses are used.

14. The use of the microcapsules claimed in claim 1 for the production of an anti-acne medicament.

## Revendications

1. Microcapsules présentant un diamètre moyen allant de 0,0001 à 5 mm, constituées d'une membrane enveloppe et d'une matrice contenant les principes actifs et pouvant être obtenues par
(a1) préparation d'une matrice à base de gélifiants, de chitosanes et d'acide glycyrrhétinique et/ou de ses dérivés,
(a2) dispersion éventuelle de la matrice dans une phase huileuse,
(a3) traitement de la matrice éventuellement en dispersion avec des solutions aqueuses de polymères anioniques et, élimination éventuelle de la phase huileuse,
ou
(b1) préparation d'une matrice à base de gélifiants, de polymères anioniques et d'acide glycyrrhétinique et/ou de ses dérivés,
(b2) dispersion éventuelle de la matrice dans une phase huileuse,
(b3) traitement de la matrice éventuellement en dispersion avec des solutions aqueuses de chitosanes et, élimination éventuelle de la phase huileuse,
ou
(c1) traitement de préparations aqueuses d'acide glycyrrhétinique et/ou de ses dérivés avec des corps huileux en présence d'émulsifiants pour obtenir des émulsions H/E,
(c2) traitement des émulsions ainsi obtenues avec des solutions aqueuses de polymères anioniques,
(c3) mise en contact de la matrice ainsi obtenue avec des solutions aqueuses de chitosanes, et
(c4) séparation des produits d'encapsulation ainsi obtenus et de la phase aqueuse.

2. Procédé de préparation de microcapsules présentant un diamètre moyen allant de 0,0001 à 5 mm, constituées d'une membrane enveloppe et d'une matrice contenant les principes actifs, selon lequel on effectue :
(a1) la préparation d'une matrice à base de gélifiants, de chitosanes et d'acide glycyrrhétinique et/ou de ses dérivés,
(a2) la dispersion éventuelle de la matrice dans une phase huileuse,
(a3) le traitement de la matrice éventuellement en dispersion avec des solutions aqueuses de polymères anioniques et, l'élimination éventuelle de la phase huileuse,
ou
(b1) la préparation d'une matrice à base de gélifiants, de polymères anioniques et d'acide glycyrrhétinique et/ou de ses dérivés,
(b2) la dispersion éventuelle de la matrice dans une phase huileuse,
(b3) le traitement de la matrice éventuellement en dispersion avec des solutions aqueuses de chitosanes et, l'élimination éventuelle de la phase huileuse,
ou
(c1) le traitement de préparations aqueuses d'acide glycyrrhétinique et/ou de ses dérivés avec des corps huileux en présence d'émulsifiants pour obtenir des émulsions H/E,
(c2) le traitement des émulsions ainsi obtenues avec des solutions aqueuses de polymères anioniques,
(c3) la mise en contact de la matrice ainsi obtenue avec des solutions aqueuses de chitosanes, et
(c4) la séparation des produits d'encapsulation ainsi obtenus et de la phase aqueuse.

3. Procédé selon la revendication 2,
**caractérisé en ce que**
on met en oeuvre des dérivés de l'acide glycyrrhétinique choisis dans le groupe constitué par les sels alcalins, les sels alcalino-terreux, les sels d'ammonium, les sels d'alkylammonium, les sels d'alcanolammonium, les sels de glucammonium et le sels de zinc de l'acide glycyrrhétinique, par les esters de l'acide glycyrrhétinique et d'alcools aliphatiques linéaires ou ramifiés comptant de 1 à 18 atomes de carbone ainsi que par les esters complets ou partiels de l'acide glycyrrhétinique et de polyols comptant de 2 à 15 atomes de carbone et au moins 2 groupes hydroxyle.

4. Procédé selon les revendications 2 et/ou 3,
**caractérisé en ce que**
en tant que dérivés de l'acide glycyrrhétinique on met en oeuvre un extrait de *Glycyrrhiza glabra.*

5. Procédé selon l'une au moins des revendications 2 à 4,
**caractérisé en ce que**
en tant que dérivés de l'acide glycyrrhétinique on met en oeuvre un sel de potassium, un sel d'ammonium et/ou un sel de zinc ou les esters de celui-ci et d'alcools gras comportant de 16 à 18 atomes de carbone.

6. Procédé selon l'une au moins des revendications 2 à 5,
**caractérisé en ce que**
en tant qu'autres principes actifs on met en oeuvre des extraits végétaux et/ou la vitamine B6.

7. Procédé selon la revendication 6,
**caractérisé en ce que**
on met en oeuvre des extraits végétaux choisis dans le groupe constitué par *Aesculus hippocastanum* (Marron d'Inde), *Argania spinosa, Baptisia tinctoria* (Indigo sauvage), *Centella asiatica, Camellia sinensis* (Thé vert), *Chamomilla recutita* (Camomille), *Ginkgo biloba* (Ginkgo), *Oleo europaea* (Olive), *Litchi chinensis* (Litchi), *Melissa officinalis* (Mélisse), *Panax ginseng* (Ginseng), *Passiflora incarnata* (Passiflore), *Prunus dulcis* (Amande douce), *Pterocarpus marsupium, Ruscus aculeatus, Trifolium pratense* (Trèfle violet), *Uva ursi* (Busserole), *Vaccinium myrtillus* (Myrtille), *Vigna aconitifolia, et Vitis vinifera* (Vigne rouge) ainsi que leurs mélanges.

8. Procédé selon les revendications 6 et/ou 7,
**caractérisé en ce que**
on met en oeuvre l'acide glycyrrhétinique et/ou ses dérivés et les extraits végétaux et/ou la vitamine B6 dans un rapport pondéral allant de 90 : 10 à 10:90.

9. Procédé selon l'une au moins des revendications 2 à 8,
**caractérisé en ce que**
en tant que gélifiant on met en oeuvre des hétéropolysaccharides ou des protéines.

10. Procédé selon la revendication 9,
**caractérisé en ce que**
en tant que hétéropolysaccharides on met en oeuvre des agaroses, de l'agar-agar, des pectines, des xanthanes ainsi que leurs mélanges.

11. Procédé selon la revendication 9,
**caractérisé en ce que**
en tant que protéines employées on met en oeuvre de la gélatine.

12. Procédé selon l'une au moins des revendications 2 à 11,
**caractérisé en ce que**
on met en oeuvre des chitosanes qui présentent un poids moléculaire moyen allant de 10 000 à 500 000 ou de 800 000 à 1 200 000 daltons.

13. Procédé selon l'une au moins des revendications 2 à 12,
**caractérisé en ce que**
on met en oeuvre des polymères anioniques choisis dans le groupe constitué par les sels de l'acide alginique, les dérivés anioniques du chitosane, les poly(méth)acrylates et les carboxyméthylcelluloses.

14. Utilisation de microcapsules selon la revendication 1, pour la préparation d'un médicament permettant de lutter contre l'acné.
